# EUROPEAN PATENT SPECIFICATION

(11) **EP 2 740 471 B1**
(45) Date of publication and mention of the grant of the patent: **27.05.2015**
(21) Application number: 12008196.3
(22) Date of filing: 07.12.2012
(51) Int. Cl.: A61K 9/16

(54) **Oral pharmaceutical composition comprising dabigatran etexilate**
Orale pharmazeutische Zusammensetzung mit Dabigatranetexilat
Composition pharmaceutique orale comprenant le dabigatran etexilate

(43) Date of publication of application: 11.06.2014
(73) Proprietor: Hexal AG, 83607 Holzkirchen (DE)
(72) Inventor: Krekeler, Andreas, 83607 Holzkirchen (DE); Göktas, Michael, 83607 Holzkirchen (DE); Wawra, Christian, 83607 Holzkirchen (DE); Neumann, Dimitri, 83607 Holzkirchen (DE)
(74) Representative: Kluschanzoff, Harald

(56) References cited:
- WO-A1-2011/107427
- WO-A1-2012/162492
- US-A1- 2003 181 488

## Description

The present invention relates to an oral pharmaceutical composition comprising dabigatran etexilate or a pharmaceutically acceptable salt thereof, methods for preparing it and dosage forms suitable for oral administration comprising said composition. The pharmaceutical composition is particularly useful as a medicament, especially as anticoagulant.

### Background prior art

Dabigatran etexilate ethyl 3-{[(2-{[(4-{N'-hexyloxycarbonyl carbamimidoyl}phenyl)amino]methyl}-1- methyl-1H-benzimidazol-5-yl)carbonyl] (pyridin-2-yl-amino)propanoate with the following structural formula I, 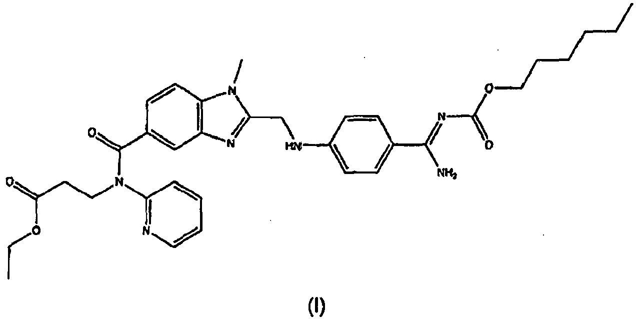 is a double prodrug that was disclosed in WO 1998/037075. The main indication field of said compound is the post-operative prevention of formation of blood cloths in the veins.

The solubility of dabigatran etexilate in water is 1.8 mg/mL and dependent on the pH value. Although dabigatran etexilate is more soluble at lower pH, it is also less stable in acidic environment. To increase the solubility of dabigatran, WO 2003/074056 suggests pharmaceutically acceptable salts of dabigatran etexilate with a water solubility of more than 1 g per 250 mL at 20 °C. In addition, in WO 2003/074056 a pharmaceutical multiparticulate pellet composition is also disclosed comprising a core comprising a pharmaceutically acceptable organic acid and a layer comprising dabigatran etexilate or a salt thereof, wherein the core and the layer comprising dabigatran etexilate are separated by an isolating layer.

WO 2011/107427 discloses an oral pharmaceutical composition comprising dabigatran etexilate or a pharmaceutically acceptable salt thereof, and an inorganic acidic excipient that have a pH value in a 1% aqueous solution of less than 6.

WO 2012/001156 discloses the process for the preparation of an oral dosage form comprising dabigatran etexilate or a salt thereof comprising a spherical core comprising tartaric acid coated with a layer comprising dabigatran etexilate, and a dosage form obtainable by said process.

There is an unmet need for oral pharmaceutical compositions comprising dabigatran etexilate that provide a fast dissolution of the drug at pH lower than 7 and an improved process for production thereof.

### Detailed description of the invention

The object of the present invention was to provide an improved pharmaceutical composition comprising dabigatran etexilate or a pharmaceutically acceptable salt thereof, as well as a more robust, economical and acceptable process for preparing said composition.

In one aspect the present invention provides a composition comprising
a spherical core comprising an inorganic acid or a salt or a hydrate thereof and at least one pharmaceutically acceptable excipient,
a first layer comprising or consisting of at least one anti-tacking agent and at least one water-soluble polymer,
a second layer comprising dabigatran etexilate or a pharmaceutically acceptable salt thereof, wherein the spherical core and the second layer are separated by the first layer.

It was surprisingly found that such a composition exhibits an improved dissolution of the drug, compared to the prior art compositions. Surprisingly, the high dissolution of dabigatran from the composition of the present Invention was achieved in a very short time frame after being dissolved in water. It was also demonstrated that the dissolution of the composition according to the present invention was even better at pH lower than 7, such as pH 5 and pH 2. The composition according to the present invention showed the highest dissolution at low pH, such as pH 2, which is in particular convenient as high dissolution is achieved at stomach pH.

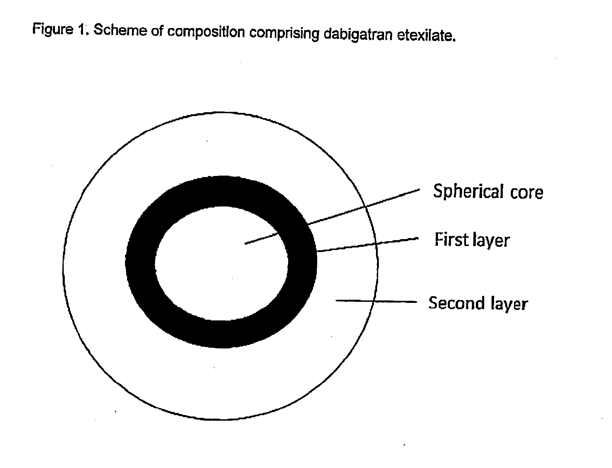

Figure 1 shows the structure of the composition according to present invention.

The term "inorganic acid" used herein is defined as an acid derived from one or more inorganic compounds. In a preferred embodiment the inorganic acid is selected from sulfuric acid, sulfonic acid, hydrochloric acid, and phosphoric acid. In another preferred embodiment the inorganic acid is sulfonic or sulfuric acid.

The inorganic acids can also be in a form of salt. Suitable inorganic acid salts are selected from alkali, earth alkali or ammonium salts of inorganic acids, in particular sodium, potassium, magnesium, calcium inorganic and ammonium acid salts. Particularly suitable inorganic acid salts are selected from sodium sulfate, magnesium sulfate, calcium sulfate, potassium sulfate, ammonium sulfate, sodium sulfonate, magnesium sulfonate, potassium sulfonate, calcium sulfonate, ammonium sulfonate, sodium chloride, magnesium chloride, potassium chloride, calcium chloride, ammonium chloride, ferrous chloride, ferric chloride, sodium phosphate, magnesium phosphate, potassium phosphate, calcium phosphate and ammonium phosphate. In a preferred embodiment the inorganic acid salt is alkali hydrogen sulfate. Particularly preferred is sodium hydrogen sulfate.

The inorganic acid salts can be present in a solvate form, in particular in a hydrate form.

In a preferred embodiment the spherical core comprises at least 40 wt % of inorganic acid. Preferably, the spherical core comprises from 40 to 80 wt % of inorganic acid, more preferably from 50 to 70 wt %. In particular, the spherical core comprises from 55 to 65 wt % of inorganic acidor salts thereof as defined above.

The term "spherical core" used herein means a core having a shape approximating that of a sphere. Preferably spherical cores have a narrow size distribution. In a preferred embodiment the size of spherical core is from 200 to 1000 µm, preferably from 400 to 800 µm, more preferably from 500 to 700 µm.

In one embodiment of the present invention the spherical core comprises a mixture of inorganic acid as defined above and a pharmaceutically acceptable excipient.

In another embodiment of the present invention the spherical core comprises a pharmaceutically acceptable excipient coated with the inorganic acid as defined above.

In a preferred embodiment the at least one pharmaceutically acceptable excipient is selected from cellulose derivatives (e.g. hypromellose, hydroxypropylcellulose, methylcellulose and sodium carboxymethylcellulose), polyvinylpyrrolidone, gelatin, lactose, sucrose, acacia, polyethylene glycol, polymethacrylates, hydroxypropylcellulose, pregelatinized starch and sodium alginate. In a preferred embodiment the pharmaceutically acceptable excipient is selected from microcrystalline cellulose, sucrose, hydroxypropylcellulose, hydroxypropylmethylcellulose and polyvinylpyrrolidone. Particularly preferred is microcrystalline cellulose.

According to the present invention, the first layer is separating the spherical core and the second layer. The first layer comprises or consists of at least one water-soluble polymer and at least one anti-tacking agent.

The term "water-soluble polymer" used herein is defined as a polymer that will have a characteristic of being substantially soluble in water. Preferably, water-soluble polymer has solubility in water determined by a weight % percentage of dissolution in water of 3.3 wt % or more, more suitably 5 wt % or more, even more suitably 10 wt % or more, particularly suitably 50 wt % or more; or which can achieve water permeability by swelling or salt formation. Suitable water-soluble polymers are selected from the group of polyethylene glycol 2000 - 6000, polyvinylpyrrolidone, polyvinyl alcohol-polyethylene glycol graft copolymer, polyoxyethylene copolymers, polyoxypropylene copolymers, polyethyleneoxide, hydroxypropyl cellulose, hydroxyethyl cellulose, hydroxyalkyl alkali metal carboxyalkylcellulose derivatives, hydroxyethyl carboxymethyl cellulose, hydroxymethyl carboxyethyl cellulose, hydroxymethyl carboxymethyl cellulose, hydroxypropyl carboxymethyl cellulose, hydroxypropyl carboxyethyl cellulose, hydroxypropyl carboxyethyl cellulose, hydroxypropyl carboxypropyl cellulose, hydroxybutyl carboxymethyl cellulosepolyethyleneoxide, carrageenan, agar, alginic acid, polymethacrylate, polyvinylacetate, dextranes, cellulose ethers and esters like methylcellulose, ethytcelfulose, methylethylcellulose, hydroxyethylcellulose, hydroxypropylmethyl cellulose, hydroxypropyl cellulose, carboxymethylcellulose; or a mixture of any of these compounds. In a preferred embodiment the water-soluble polymer is selected from hydroxypropylmethylcellulose, hydroxypropylcellulose and polyvinylpyrrolidone.

"Anti-tacking agent" used herein means compounds able to reduce or prevent adhesion between particles. Anti-tacking agent may be present in the pharmaceutical composition in the form of a single compound or in the form of a mixture of compounds. Various anti-tacking agents are suitable, including hydrated earth alkali silicates, magnesium stearate, calcium stearate and sodium stearyl fumarate. In a preferred embodiment the anti-tacking agent is selected from hydrated earth alkali silicates, preferably talc.

In another preferred embodiment, the spherical core comprises alkali hydrogen sulfate and microcrystalline cellulose and the first layer comprises or consists of talc and polyvinylpyrrolidone or talc and hydroxypropylmethylcellulose.

In another preferred embodiment the mass ratio of water-soluble polymer : anti-tacking agent is 0.5 : 1. Preferably, the mass ratio of water-soluble polymer : anti-tacking agent is 0.8 : 1, more preferably 1:1.

In another preferred embodiment the thickness of the first layer is from 5 to 40 µm, preferably from 10 to 30 µm.

The second layer comprises dabigatran etexilate or a pharmaceutically acceptable salt thereof. In a preferred embodiment the second layer further comprises at least one at least one water-soluble polymer and an anti-tacking agent. Suitable water-soluble polymers and anti-tacking agents are stated above. In the most preferred embodiment, the second layer further comprises hydroxypropylcellulose and talc.

In another aspect the present invention further comprises at least one controlled-release polymer. The term "controlled release polymer", or its synonymous term "release-rate controlling polymer", refers to a polymer, on account of which a modified release, a retarded/delayed release, a prolonged release or a pulsatile release of the dabigatran active ingredient the pharmaceutical composition is accomplished, relative to a direct or immediate release (also sometimes called conventional release) defined by unaffected dissolution under a given condition. Suitable controlled release polymers include, without being limited thereto, polyvinylpyrrolidone, polymethacrylate, polyvinylacetate, dextranes, starch, cellulose ethers and esters like methyl cellulose, methylethylcellulose, hydroxyethylcellulose, hydroxypropylmethylcellulose, hydroxypropylcellulose, or carboxymethylcellulose, polyethyleneoxide, carrageenan, agar, alginic acid, pectin or mixtures thereof, wherein where applicable controlled release types of a specified polymer, as opposed to immediate release types, are selected. It is preferably that the controlled release polymer is present in an amount that allows for the formation of a gel matrix or that allows a the active ingredient to be suitably released in a controlled manner, more preferably by gradual release. According to a further preferred embodiment, the controlled release polymer is Eudragit or ethyl cellulose.

In a preferred embodiment controlled release polymer is applied as an over-layer on the second layer comprising dabigatrane etexilate or a pharmaceuticall acceptable salt thereof. Alternatively, controlled release polymer is comprised in the second layer of the present invention.

In a preferred embodiment the pharmaceutical composition according to the present invention further comprises an over-coat. The term "over-coat" used herein refers to a layer which completely covers an object and is applied by film coating. The over-coat can be selected from the group of ready to form dispersion such as OPADRY. The coating dispersion comprises hydrophilic film forming polymer (such as for example low viscosity HPMC, HPC, PVA (polyvinylalcohol) and the like), plastificators (e.g. PEG), colorants and may optionally include other excipients such as anti-tacking agents.

In another aspect the present invention relates to a capsule comprising one or more units of the pharmaceutical composition of the present invention. Said capsules usually consist of the material selected from hydroxypropylmethylcellulose or gelatine. Further additives like carrageenan, potassium chloride, titanium dioxide and colorants may be present in the capsule shell.

Surprisingly, it was found that the capsules comprising one or more units of the composition comprising dabigatran etexllate or a pharmaceutically acceptable salt thereof according to the present invention showed an improved dissolution of dabigatran etexilate. In particular, the dissolution was even better at pH lower than 7, such as pH 5 and pH 2.

The capsules may be packed into a primary packaging having decreased permeability for water vapor. Suitable packaging is selected from high density polyethylene containers, polypropylene containers, aluminum foil blisters and polychlorotriflouroethylene blisters.

In another aspect the present invention the composition according to the present invention are compressed into tablets. In a preferred embodiment the composition according to the present invention further comprises pharmaceutically acceptable excipient selected from the group fillers, binders, lubricants, glidants and disintegrants.

The pharmaceutical compositions described herein can further contain fillers such as microcrystalline cellulose, powdered cellulose, compressible sugar, starch (e.g., corn starch or potato starch), pregelatinized starch, fructose, mannitol, dextranes, other sugars such as, siliconized microcrystalline cellulose, calcium hydrogen phosphate, calcium hydrogen phosphate dihydrate, tricalciumphosphate, calcium lactate or mixtures thereof. The fillers may be present in the form of a single compound or in the form of a mixture of compounds or co-processed compounds.

The compositions described herein may also comprise binders, such as cellulose derivatives (e.g. hypromellose, hydroxypropylcellulose, methylcellulose and sodium carboxymethylcellulose), polyvinylpyrrolidone, gelatin, lactose, sucrose, acacia, polyethylene glycol, polymethacrylates, hydroxypropylcellulose, pregelatinized starch and sodium alginate. The term "binder" as used herein is defined as an agent able to bind particles which cannot be bound only by a compression force. The binder may be present in the form of a single compound or in the form of a mixture of compounds.

The compositions described herein may also comprise lubricants. Various suitable lubricants include but are not limited to stearic acid, talc, hydrogenated vegetable oil (e.g. hydrogenated castor oil), sodium lauryl sulphate, glyceryl behenate, polyethylene glycol, magnesium stearate, calcium stearate and sodium stearyl fumarate.

The term "glidant" used herein is defined as an agent improving the flow of the powder and thus the filling of the compression chamber of the tablet press. Glidant may be present in the pharmaceutical composition in the form of a single compound or in the form of a mixture of compounds. In a preferred embodiment the glidant is selected from hydrated earth alkali silicates, preferably talc.

The compositions described herein may also comprise disintegrants. The term disintegrant as used herein is an agent accelerating the disintegration of the composition when in contact with a liquid. Preferred disintegrants are polyvinylpyrrolidone, crosslinked polyvinylpyrrolidone (crospovidone), crosslinked sodium carboxymethyl cellulose, sodium carboxymethyl starch, sodium carboxymethyl glycolate and sodium bicarbonate.

In yet another aspect, the present invention relates to a process for preparation of an oral pharmaceutical composition comprising dabigatran etexilate. In a preferred embodiment the process for preparation of an oral pharmaceutical composition comprises the steps of
providing the spherical core comprising acid or a salt or a hydrate thereof and at least one pharmaceutically acceptable excipient,
applying the first layer on the surface of the spherical core,
applying the second layer on the first layer.

It was surprisingly found that the composition comprising dabigatran etexilate can be prepared in a simplified and robust manner. The process according to the present invention provided a simple and reliable method for the preparation of compositions comprising dabigatran etexilate or a pharmaceutically acceptable salt thereof. Unexpectedly, the compositions prepared according to the process of the present invention have a regular shape and uniform size distribution.

In a preferred embodiment the spherical core is obtained by homogenously mixing the inorganic acid or a salt or a hydrate thereof and at least one pharmaceutically acceptable excipient.

In another preferred embodiment the spherical core is obtained by providing at least one pharmaceutically acceptable excipient, coating the excipient with the inorganic acid or a salt or a hydrate thereof.

In another preferred embodiment the coating of the pharmaceutically acceptable excipient is performed by fluid bed process. In another preferred embodiment the coating of the pharmaceutically acceptable excipient is performed by extrusion/spheronization process.

Furthermore, it was surprising found that the spherical cores obtained by the process for preparation according to the present invention are regularly shaped.

In yet another aspect the present invention relates to an oral pharmaceutical composition comprising a regularly shaped spherical core obtained by homogenous mixing of the inorganic acid or a salt or a hydrate thereof and at least one pharmaceutically acceptable excipient.

In another aspect the present invention relates to an oral pharmaceutical composition comprising a regularly shaped spherical core obtained by providing at least one pharmaceutically acceptable excipient, coating the excipient with the inorganic acid or salt or a hydrate thereof.

In another aspect the present invention relates to an oral pharmaceutical composition for use in the treatment and/or prevention of blood coagulation and thrombotic events, such as prevention of venous thromboembolism.

### Examples

### Example 1

| Component | Amount per capsule (mg) |
|---|---|
| **Composition** | |

| Core | |
|---|---|
| Sodium hydrogen sulfate (NaHSO₄) | 91.26 |
| Microcrystalline cellulose (MCC) (Avicel PH101) | 39.11 |

| Isolating layer | |
|---|---|
| Polyvinylpyrrolidone (Kollidon 25) | 16.76 |
| Talc | 39.12 |

| API layer | |
|---|---|
| Dabigatran Etexilate Mesylate | 172.97 |
| Hydroxypropylcellulose (HPC) (Klucel EF) | 39.74 |
| Talc | 21.04 |
| **Sum** | 420.0 |

| **Capsule shell** | |
|---|---|
| Hydroxypropylmethylcellulose (HPMC) capsule size 0 | |

The process for preparation of the pellets according to Example 1

**Core:** Sodium hydrogen sulfate and MCC were mixed in a suitable mixer. The mixture was wetted by a suitable amount of water and the mass afterwards was extruded and spheronized with aid of an extruder / spheronizer (NICA system). The cores were dried at 25°C for 24h. **Intermediate layer:** Kollidon was dissolved in ethanol. Afterwards talc was suspended in this solution. The suspension was sprayed onto the cores by a fluid bed coating system (type Wurster).

**API layer:** HPC was added to isopropanol and stirred until dissolved. Afterwards talc was added and stirred. Finally, the active ingredient was added, stirred and the suspension was homogenized with a suitable device. The suspension was sprayed onto the cores with intermediate layer by a fluid bed coating system (type Wurster).

### Example 2

| Component | Amount per capsule (mg) |
|---|---|
| **Composition** | |
| Core | |
| Sodium hydrogen sulfate (NaHSO₄) | 49.62 |
| Microcrystalline cellulose (MCC) (Avicel PH101) | 74.50 |

| Isolating layer | |
|---|---|
| Polyvinylpyrrolidone (Kollidon 25) | 21.73 |
| Talc | 40.35 |

| API layer | |
|---|---|
| Dabigatran Etexilate Mesylate | 172.97 |
| Hydroxypropylcellulose (HPC) (Klucel EF) | 39.74 |
| Talc | 21.04 |
| **Sum** | 420.0 |

| **Capsule shell** | |
|---|---|
| Hydroxypropylmethylcellulose (HPMC) capsule size 0 | |

The process for preparation of the pellets according to Example 2

**Core:** Sodium hydrogen sulfate and MCC were mixed in a suitable mixer. The mixture was wetted by a suitable amount of water and the mass afterwards was extruded and spheronized with aid of an extruder / spheronizer (NICA system). The cores were dried at 25°C for 24h. Intermediate layer: Kollidon was dissolved in ethanol. Afterwards talc was suspended in this solution. The suspension was sprayed onto the cores by a fluid bed coating system (type Wurster).

**API layer:** HPC was added to isopropanol and stirred until dissolved. Afterwards talc was added and stirred. Finally, the active ingredient was added, stirred and the suspension was homogenized with a suitable device. The suspension was sprayed onto the cores with intermediate layer by a fluid bed coating system (type Wurster).

### Example 3

| Component | Amount per capsule (mg) |
|---|---|
| **Composition** | |
| Core | |
| Microcrystalline cellulose (MCC) pellets (Cellets 500µm) | 56 |
| Sodium hydrogen sulfate (NaHSO₄) | 84 |

| Isolating layer | |
|---|---|
| Hydroxypropylmethylcellulose (HPMC) | 23.13 |
| Talc | 23.13 |

| API layer | |
|---|---|
| Dablgatran Etexilate Mesylate | 172.97 |
| Hydroxypropylcellulose (HPC) (Klucel EF) | 39.74 |
| Talc | 21.04 |
| **Sum** | 420.0 |

| **Capsule shell** | |
|---|---|
| Hydroxypropylmethylcellulose (HPMC) capsule size 0 | |

The process for preparation of the pellets according to Example 3

**Core:** An aqueous solution of sodium hydrogen sulfate was sprayed onto MCC pellets by use of a fluid bed coating system (type Wurster).

Intermediate **layer:** HPMC was dissolved in ethanol. Afterwards talc was suspended in this solution. The suspension was sprayed onto the cores by a fluid bed coating system (type Wurster).

**API layer:** HPC was added to isopropanol and stirred until dissolved. Afterwards talc was added and stirred. Finally the active ingredient was added, stirred and the suspension was homogenized with a suitable device. The suspension was sprayed onto the cores with intermediate layer by a fluid bed coating system (type Wurster).

### Comparative example

| Component | Amount per capsule (mg) |
|---|---|
| **Composition** | |
| Core | |
| Gum Arabic | 8.86 |
| Tartaric acid | 177.14 |

| Isolating layer | |
|---|---|
| Hypomellose | 4.46 |
| Dimethylpolysiloxane | 0.08 |
| Talc | 28.07 |

| API layer | |
|---|---|
| Dabigatran Etexilate Mesylate | 172.95 |
| Hydroxypropylcellulose | 34.60 |
| Talc | 6.24 |
| **Sum** | 432.4 |

| **Capsule shell** | |
|---|---|
| Hydroxypropylmethylcellulose (HPMC) capsule size 0 | |

### The measurement of dissolution

Dissolution of dabigatran etexilate was measured as the percentage of the release of dabigatran etexilate from the composition after 5 minutes in the individual dissolution medium.

Dissolution of dabigatran etexilate was measured with the following methods:

### Method 1

A suitable amount of dabigatran etexilate composition was transferred to 900 mL 0.01 M hydrochloric acid pH 2. An agitation speed of 75 rpm in the USP II apparatus was used for dissolution. Samples were measured UV photometric after 5 minutes, using an appropriate wavelength.

### Method 2

A suitable amount of dabigatran etexilate composition was transferred to 500 mL 0.005 M potassium dihydrogen phosphate buffer pH 5. An agitation speed of 75 rpm in the USP II apparatus was used for dissolution. Samples were measured UV photometric after 5 minutes, using an appropriate wavelength.

| | Dissolution at pH 2 (%) | Dissolution at pH 5 (%) |
|---|---|---|
| Composition of Example 1 | 74 | 32 |
| Composition of Example 2 | 80 | 27 |
| Composition of Example 3 | 58 | 30 |
| Composition of Comparative example | 54 | 13 |

The aspects, advantageous features and preferred embodiments of the present invention summarized in the following items, respectively alone or in combination, further contribute to solving the object of the invention:
1. An oral pharmaceutical composition comprising
   a spherical core comprising an inorganic acid or a salt or a hydrate thereof and at least one pharmaceutically acceptable excipient,
   a first layer comprising or consisting of at least one water-soluble polymer and at least one anti-tacking agent,
   a second layer comprising dabigatran etexilate or a pharmaceutically acceptable salt thereof,
   wherein the spherical core and the second layer are separated by the first layer.
2. The oral pharmaceutical composition according to item 1, wherein the spherical core comprises a mixture of inorganic acid or a salt or a hydrate thereof and the pharmaceutically acceptable excipient.
3. The oral pharmaceutical composition according to item 1, wherein the spherical core comprises the pharmaceutically acceptable excipient coated with the inorganic acid or a salt or a hydrate thereof.
4. The oral pharmaceutical composition according to item 1, wherein the inorganic acid is selected from sulfuric acid, sulfonic acid, hydrochloric acid, and phosphoric acid or a salt or a hydrate thereof.
5. The oral pharmaceutical composition according to any of the preceding items, wherein the inorganic acid is sulfonic acid or a salt or a hydrate thereof.
6. The oral pharmaceutical composition according to any of the preceding items, wherein the inorganic acid is sulfuric acid or a salt or a hydrate thereof.
7. The oral pharmaceutical composition according to any of the preceding items, wherein the inorganic acid salt is alkali hydrogen sulfate.
8. The oral pharmaceutical composition according to any of the preceding items, wherein the inorganic acid salt is sodium hydrogen sulfate.
9. The oral pharmaceutical composition according to any of the preceding items, wherein the spherical core comprises at least 40 wt % of inorganic acid or a salt or a hydrate thereof.
10. The oral pharmaceutical composition according to any of the preceding items, wherein the spherical core comprises from 40 to 80 wt % of inorganic acid or a salt or a hydrate thereof.
11. The oral pharmaceutical composition according to any of the preceding items, wherein the spherical core comprises from 50 to 70 wt % of inorganic acid or a salt or a hydrate thereof.
12. The oral pharmaceutical composition according to any of the preceding items, wherein the spherical core comprises from 55 to 65 wt % of inorganic acid or a salt or a hydrate thereof.
13. The oral pharmaceutical composition according to any of the preceding items, wherein the size of spherical core is from 200 to 1000 µm.
14. The oral pharmaceutical composition according to any of the preceding items, wherein the size of spherical core is 400 to 800 µm.
15. The oral pharmaceutical composition according to any of the preceding items, wherein the size of spherical core is from 500 to 700 µm.
16. The oral pharmaceutical composition according to any of the preceding items, wherein the at least one pharmaceutically acceptable excipient is selected from microcrystalline cellulose, sucrose, hydroxypropylcellulose, hydroxypropylmethylcellulose and polyvinylpyrrolidone.
17. The oral pharmaceutical composition according to any of the preceding items, wherein the water-soluble polymer is selected from hydroxypropylmethylcellulose, hydroxypropylcellulose and polyvinylpyrrolidone.
18. The oral pharmaceutical composition according to any of the preceding items, wherein the anti-tacking agent is selected from hydrated earth alkali silicates.
19. The oral pharmaceutical composition according to any of the preceding items, wherein the anti-tacking agent is talc.
20. The oral pharmaceutical composition according to any of the preceding items, wherein the mass ratio of water-soluble polymer: anti-tacking agent is 0.5 : 1.
21. The oral pharmaceutical composition according to any of the preceding items, wherein the mass ratio of water soluble polymer: anti-tacking agent is 0.8 : 1.
22. The oral pharmaceutical composition according to any of the preceding items, wherein the mass ratio of water-soluble polymer: anti-tacking agent is 1 : 1.
23. The oral pharmaceutical composition according to any of the preceding items, wherein the thickness of the first layer is from 5 to 40 µm.
24. The oral pharmaceutical composition according to any of the preceding items, wherein the thickness of the first layer is from 10 to 30 µm.
25. The oral pharmaceutical composition according to any of the preceding items further comprising an extended release polymer.
26. The oral pharmaceutical composition according to any of the preceding items further comprising an over-coat.
27. The oral pharmaceutical composition according to item 1, wherein the spherical core comprising alkali hydrogen sulfate and microcrystalline cellulose and the first layer comprising or consisting of talc and hydroxypropylcellulose.
28. The oral pharmaceutical composition according to item 1, wherein the spherical core comprising alkali hydrogen sulfate and microcrystalline cellulose and the first layer comprising or consisting of talc and polyvinylpyrrolidone.
29. A capsule comprising one or more units of the pharmaceutical composition according to any of the preceding items.
30. A process for preparation of an oral pharmaceutical composition according to any of items 1 to 28, comprising the steps of
   providing the spherical core comprising inorganic acid or a salt or a hydrate thereof and at least one pharmaceutically acceptable excipient,
   applying the first layer as defined in item 1 on the surface of the spherical core,
   applying the second layer as defined in item 1 on the first layer.
31. The process according to item 30, wherein the spherical core is obtained by homogenously mixing the inorganic acid or a salt or a hydrate thereof and at least one pharmaceutically acceptable excipient.
32. The process according to item 30, wherein the spherical core is obtained by coating the excipient with the inorganic acid or a salt or a hydrate thereof.
33. The process according to item 32, wherein the coating of the pharmaceutically acceptable excipient is performed by fluid bed process.
34. The process according to item 32, wherein the coating of the pharmaceutically acceptable excipient is performed by extrusion/spheronization process.
35. An oral pharmaceutical composition comprising a regularly shaped spherical core according to item 1 obtained by the process for preparation according to item 30.
36. An oral pharmaceutical composition comprising a regularly shaped spherical core according to item 1 obtained by the process for preparation according to item 31.
37. An oral pharmaceutical composition comprising a regularly shaped spherical core according to item 1 obtained by the process for preparation according to items 32 to 34.
38. The composition according to any of the preceding items 1 to 28 for use in the treatment and/or prevention of blood coagulation and thrombotic events.

## Claims

1. An oral pharmaceutical composition comprising
a spherical core comprising an inorganic acid or a salt or a hydrate thereof and at least one pharmaceutically acceptable excipient,
a first layer comprising or consisting of at least one water-soluble polymer and at least one anti-tacking agent,
a second layer comprising dabigatran etexilate or a pharmaceutically acceptable salt thereof,
wherein the spherical core and the second layer are separated by the first layer.

2. The oral pharmaceutical composition according to claim 1, wherein the inorganic acid is selected from sulfuric acid, sulfonic acid, hydrochloric acid, and phosphoric acid or a salt or a hydrate thereof.

3. The oral pharmaceutical composition according to any of the preceding claims, wherein the inorganic acid is sulfonic and/or sulfuric acid or a salt or a hydrate thereof, preferably alkali hydrogen sulfate, more preferably sodium hydrogen sulfate.

4. The oral pharmaceutical composition according to any of the preceding claims, wherein the spherical core comprises at least 40 wt % of inorganic acid or a salt or a hydrate thereof, preferably from 40 to 80 wt %, more preferably from 50 to 70 wt %, in particular from 55 to 65 wt %.

5. The oral pharmaceutical composition according to any of the preceding claims, wherein the size of spherical core is from 200 to 1000 µm, preferably from 400 to 800 µm, more preferably from 500 to 700 µm.

6. The oral pharmaceutical composition according to any of the preceding claims, wherein the at least one pharmaceutically acceptable excipient is selected from microcrystalline cellulose, sucrose, hydroxypropylcellulose, hydroxypropylmethylcellulose and polyvinylpyrrolidone.

7. The oral pharmaceutical composition according to any of the preceding claims, wherein the water-soluble polymer is selected from hydroxypropylmethylcellulose, hydroxypropylcellulose and polyvinylpyrrolidone.

8. The oral pharmaceutical composition according to any of the preceding claims, wherein the anti-tacking agent is selected from hydrated earth alkali silicates, preferably talc.

9. The oral pharmaceutical composition according to any of the preceding claims, wherein the mass ratio of water-soluble polymer: anti-tacking agent is 0.5 : 1, preferably 0.8 : 1, more preferably 1:1.

10. The oral pharmaceutical composition according to any of the preceding claims further comprising an over-coat.

11. A capsule comprising one or more units of the pharmaceutical composition according to any of the preceding claims.

12. A process for preparation of an oral pharmaceutical composition according to any of claims 1 to 10, comprising the steps of
providing the spherical core comprising an inorganic acid or a salt or a hydrate thereof and at least one pharmaceutically acceptable excipient,
applying the first layer as defined in claim 1 on the surface of the spherical core,
applying the second layer as defined in claim 1 on the first layer.

13. The process according to any of claim 12, wherein the spherical core is obtained by homogenously mixing the inorganic acid or a salt or a hydrate thereof and at least one pharmaceutically acceptable excipient, or coating the pharmaceutically acceptable excipient with the inorganic acid or a salt or a hydrate thereof.

14. An oral pharmaceutical composition comprising a regularly shaped spherical core according to claim 1 obtained by the process for preparation according to claim 12.

15. The oral composition according to any of the preceding claims 1 to 10 and 14 for use in the treatment and/or prevention of blood coagulation and thrombotic events.

## Patentansprüche

1. Orale pharmazeutische Zusammensetzung umfassend
einen sphärischen Kern, welcher eine anorganische Säure oder ein Salz oder ein Hydrat davon und mindestens einen pharmazeutisch akzeptablen Exzipienten umfasst,
eine erste Schicht, welche mindestens ein wasserlösliches Polymer und mindestens ein Antiklebemittel umfasst oder daraus besteht,
eine zweite Schicht, welche Dabigatranetexialt oder ein pharmazeutisch akzeptables Salz davon umfasst,
wobei der sphärische Kern und die zweite Schicht durch die erste Schicht voneinander getrennt sind.

2. Orale pharmazeutische Zusammensetzung nach Anspruch 1, wobei die anorganische Säure aus Schwefelsäure, Sulfonsäure, Chlorwasserstoffsäure, und Phosphorsäure oder einem Salz oder einem Hydrat davon ausgewählt ist.

3. Orale pharmazeutische Zusammensetzung nach einem der vorangehenden Ansprüche, wobei die anorganische Säure Schwefelsäure und/oder Sulfonsäure oder ein Salz oder ein Hydrat davon, vorzugsweise Alkalihydrogensulfat und weiter bevorzugt Natriumhydrogensulfat, ist.

4. Orale pharmazeutische Zusammensetzung nach einem der vorangehenden Ansprüche, wobei der sphärische Kern mindestens 40 Gew. % anorganische Säure oder ein Salz oder ein Hydrat davon, vorzugsweise von 40 bis 80 Gew. % und weiter bevorzugt von 50 bis 70 Gew. %, und insbesondere von 55 bis 65 Gew. % umfasst.

5. Orale pharmazeutische Zusammensetzung nach einem der vorangehenden Ansprüche, wobei die Große des sphärischen Kerns 200 bis 1000 µm, vorzugsweise 400 bis 800 µm und weiter bevorzugt 500 bis 700 µm beträgt.

6. Orale pharmazeutische Zusammensetzung nach einem der vorangehenden Ansprüche, wobei der mindestens eine pharmazeutisch akzeptable Exzipient aus mikrokristalliner Cellulose, Saccharose, Hydroxypropylcellulose, Hydroxypropylmethylcellulose und Polyvinylpyrrolidon ausgewählt ist.

7. Orale pharmazeutische Zusammensetzung nach einem der vorangehenden Ansprüche, wobei das wasserlösliche Polymer aus Hydroxypropylmethylcellulose, Hydroxypropylcellulose und Polyvinylpyrrolidon ausgewählt ist.

8. Orale pharmazeutische Zusammensetzung nach einem der vorangehenden Ansprüche, wobei das Antiklebemittel aus hydratisierten Erdalkalisilikaten, vorzugsweise Talk, ausgewählt ist.

9. Orale pharmazeutische Zusammensetzung nach einem der vorangehenden Ansprüche, wobei das Massenverhältnis von wasserlöslichem Polymer: Antiklebemittel 0,5 : 1, vorzugsweise 0,8 : 1 und weiter bevorzugt 1 : 1 beträgt.

10. Orale pharmazeutische Zusammensetzung nach einem der vorangehenden Ansprüche, ferner umfassend einen Überzug.

11. Kapsel umfassend eine oder mehrere Einheiten der pharmazeutischen Zusammensetzung nach einem der vorangehenden Ansprüche.

12. Verfahren zur Herstellung von einer oralen pharmazeutischen Zusammensetzung nach einem der vorangehenden Ansprüche 1 bis 10, umfassend die Schritte Bereitstellung des sphärischen Kerns, welcher eine anorganische Säure oder ein Salz oder ein Hydrat davon und mindestens einen pharmazeutisch akzeptablen Exzipienten umfasst,
Aufbringen der in Anspruch 1 definierten ersten Schicht nach auf der Oberfläche des sphärischen Kerns,
Aufbringen der in Anspruch 1 definierten zweiten Schicht nach auf die erste Schicht.

13. Verfahren nach Anspruch 12, wobei der sphärische Kern durch homogenes Mischen der anorganischen Säure oder eines Salzes oder eines Hydrats davon und mindestens einem pharmazeutisch akzeptablen Exzipienten, oder durch Beschichten des pharmazeutisch akzeptablen Exzipienten mit der anorganischen Säure oder einem Salz oder einem Hydrat davon erhalten wurde.

14. Orale pharmazeutische Zusammensetzung umfassend einen gleichmäßig geformten sphärischen Kern nach Anspruch 1, welcher durch das Verfahren zur Herstellung nach Anspruch 12 erhalten wurde.

15. Orale pharmazeutische Zusammensetzung nach einem der vorangegangenen Ansprüche 1 bis 10 und 14 zur Verwendung bei der Behandlung und/oder Prävention von Blutgerinnung und thrombotischen Ereignissen.

## Revendications

1. Une composition pharmaceutique orale comprenant
un noyau sphérique comprenant un acide inorganique ou un sel ou hydrate de celui-ci, ainsi qu'au moins un excipient pharmaceutiquement acceptable,
une première couche comprenant ou consistant en au moins un polymère soluble dans l'eau et au moins un agent antiadhérent,
une deuxième couche comprenant du dabigatran étexilate ou un sel pharmaceutiquement acceptable de celui-ci,
dans laquelle le noyau sphérique et la deuxième couche sont séparés par la première couche.

2. La composition pharmaceutique orale conformément à la revendication 1, dans laquelle l'acide inorganique est choisi parmi l'acide sulfurique, l'acide sulfonique, l'acide chlorhydrique et l'acide phosphorique ou un sel ou hydrate de ceux-ci.

3. La composition pharmaceutique orale selon l'une des revendications précédentes, dans laquelle l'acide inorganique est de l'acide sulfonique et/ou sulfurique ou un sel ou hydrate de celui-ci, de préférence un hydrogénosulfate alcalin, ou préférentiellement, un hydrogénosulfate de sodium.

4. La composition pharmaceutique orale selon l'une des revendications précédentes, dans laquelle le noyau sphérique comprend au moins 40 % en poids d'acide inorganique ou sel ou hydrate de celui-ci, de préférence entre 40 et 80 % en poids, et préférentiellement, entre 50 et 70 % en poids, en particulier de 55 à 65 % en poids.

5. La composition pharmaceutique orale selon l'une des revendications précédentes, dans laquelle la dimension du noyau sphérique est comprise entre 200 et 1000 µm, de préférence entre 400 et 800 µm, et préférentiellement entre 500 et 700 µm.

6. La composition pharmaceutique orale selon l'une des revendications précédentes, dans laquelle au moins l'un des excipients pharmaceutiques acceptables est choisi parmi de la cellulose microcrystalline, de la sucrose, l'hydroxypropylcellulose, l'hydroxypropylmethylcellulose et de la polyvinylpyrrolidone.

7. La composition pharmaceutique orale selon l'une des revendications précédentes, dans laquelle le polymère soluble dans l'eau est choisi parmi l'hydroxypropylmethylcellulose, l'hydroxypropylcellulose et de la polyvinylpyrrolidone.

8. La composition pharmaceutique orale selon l'une des revendications précédentes, dans laquelle l'agent antiadhérent est choisi parmi les silicates d'alcalino-terreux hydratés, de préférence du talc.

9. La composition pharmaceutique orale selon l'une des revendications précédentes, dans laquelle le rapport de masse du polymère soluble dans l'eau : agent antiadhérent est 0,5 : 1, de préférence 0,8 : 1, et préférentiellement 1:1.

10. La composition pharmaceutique orale selon l'une des revendications précédentes, comprenant de plus une surcouche.

11. Une gélule comprenant une ou plusieurs unités de la composition pharmaceutique, selon l'une des revendications précédentes.

12. Un procédé de préparation d'une composition pharmaceutique orale selon l'une des revendications de 1 à 10, comprenant les étapes de
la mise à disposition du noyau sphérique comprenant un acide inorganique ou un sel inorganique ou encore un hydrate de sel inorganique, ainsi qu'au moins un excipient pharmaceutiquement acceptable,
l'application de la première couche telle que définie dans la revendication 1, sur la surface du noyau sphérique,
l'application de la deuxième couche telle que définie dans la revendication 1, sur la première couche.

13. Le procédé selon l'une des revendications de la section 12, dans laquelle le noyau sphérique est obtenu
par mélange homogène de l'acide inorganique ou du sel ou de l'hydrate de celui-ci et au moins un excipient pharmaceutique acceptable, ou en revêtant l'excipient pharmaceutique acceptable avec l'acide inorganique ou le sel ou l'hydrate de celui-ci.

14. Une composition pharmaceutique orale comprenant un noyau sphérique da forme régulière selon la revendication 1, obtenue à l'aide du procédé de préparation selon la revendication 12.

15. La composition orale selon l'une des revendications précédentes, 1 à 10 et 14, destinée à être utilisée dans le traitement et/ou la prévention en matière de la coagulation du sang et des troubles thrombotiques.
